(19) Europäisches Patentamt
European Patent Office
Office européen des brevets

(11) **EP 3 787 741 B1**

(12) **EUROPÄISCHE PATENTSCHRIFT**

(45) Veröffentlichungstag und Bekanntmachung des Hinweises auf die Patenterteilung:
**01.03.2023 Patentblatt 2023/09**

(21) Anmeldenummer: **19713447.1**

(22) Anmeldetag: **22.03.2019**

(51) Internationale Patentklassifikation (IPC):
**A61N 5/02** *(2006.01)* **A61B 18/18** *(2006.01)*
**A61B 5/0507** *(2021.01)* **A61B 5/0538** *(2021.01)*
**A61B 5/053** *(2006.01)* **A61B 5/00** *(2006.01)*
**A61B 17/00** *(2006.01)*

(52) Gemeinsame Patentklassifikation (CPC):
**A61N 5/02; A61B 5/0507; A61B 5/053;**
**A61B 5/0538; A61B 18/1815;** A61B 5/6848;
A61B 2017/00725; A61B 2018/1869

(86) Internationale Anmeldenummer:
**PCT/EP2019/057266**

(87) Internationale Veröffentlichungsnummer:
**WO 2019/211044 (07.11.2019 Gazette 2019/45)**

(54) **VORRICHTUNG ZUR KALIBRIERUNG EINES MIKROWELLENAPPLIKATORS**

APPARATUS FOR CALIBRATING A MICROWAVE APPLICATOR

DISPOSITIF D'ÉTALONNAGE D'UN APPLICATEUR DE MICRO-ONDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.05.2018 DE 102018110501**

(43) Veröffentlichungstag der Anmeldung:
**10.03.2021 Patentblatt 2021/10**

(73) Patentinhaber: **Technische Universität Darmstadt 64289 Darmstadt (DE)**

(72) Erfinder:
• **HESSINGER, Carolin**
**55130 Mainz (DE)**
• **JAKOBY, Rolf**
**61191 Rosbach (DE)**
• **SCHMIDT, Sönke**
**64289 Darmstadt (DE)**
• **SCHÜSSLER, Martin**
**63768 Hösbach (DE)**

(74) Vertreter: **LifeTech IP**
**Spies & Behrndt Patentanwälte PartG mbB**
**Elsenheimerstraße 47a**
**80687 München (DE)**

(56) Entgegenhaltungen:
**US-A1- 2008 234 574     US-A1- 2010 030 107**
**US-A1- 2014 046 174**

• **REIMANN CAROLIN ET AL: "Microwave Ablation Applicator with Sensing Capabilities for Thermal Treatment of Malignant Tissue", 2018 IEEE/MTT-S INTERNATIONAL MICROWAVE SYMPOSIUM - IMS, IEEE, 10. Juni 2018 (2018-06-10), Seiten 1278-1281, XP033388208, DOI: 10.1109/MWSYM.2018.8439625 [gefunden am 2018-08-17]**
• **Alessandra La Gioia ET AL: "Open-Ended Coaxial Probe Technique for Dielectric Measurement of Biological Tissues: Challenges and Common Practices", Diagnostics (Basel, Switzerland), 5. Juni 2018 (2018-06-05), Seite 40, XP055556648, Switzerland DOI: 10.3390/diagnostics8020040 Gefunden im Internet: URL:https://www.mdpi.com/2075-4418/8/2/40 [gefunden am 2019-02-14]**

**Beschreibung**

Die vorliegende Erfindung bezieht sich auf eine Vorrichtung zur Kalibrierung eines Mikrowellenapplikators und insbesondere auf ein Kalibrierungsschema zur Bestimmung von dielektrischen Eigenschaften von umgebendem Gewebe durch einen Mikrowellenapplikator.

Hintergrund

Während der Mikrowellen-Behandlung von malignem Gewebe ist es möglich, durch bildgebende Verfahren wie beispielsweise Computertomografie, Ultraschall oder Magnetresonanztomografie (MRT) die Position des Mikrowellenapplikators relativ zu dem Tumor im Patienten zu bestimmen. Jedoch ist es nur schwer möglich, die genaue Abgrenzung zwischen malignem und gesundem Gewebe zu ermitteln.

Daher besteht ein Bedarf nach Mikrowellenapplikatoren, die in der Lage sind, möglichst genau die Umrisse des malignen Gewebes festzustellen, sodass ein behandelnder Arzt sofort feststellen kann, ob sich der Mikrowellenapplikator in einem gesunden Gewebe oder einem krankhaften Gewebe befindet, um basierend darauf die Behandlung durchführen zu können.

Gesundes und malignes Gewebe unterscheiden sich in ihren dielektrischen Eigenschaften wie beispielsweise in der (komplexwertigen) Permittivität, wobei beim Übergang ein Sprung in der Permittivität zwischen 15 und 30 % zu erwarten ist. Falls die Permittivität des umgebenden, gesunden Gewebes möglichst genau gemessen wird, kann genau festgestellt werden, wann der genutzte Mikrowellenapplikator von einem gesunden in ein malignes Gewebe gelangt bzw. bis wohin das krankhafte Gewebe reicht.

Hierfür ist es vorteilhaft, wenn der Mikrowellenapplikator möglichst genau die Permittivität innerhalb von Gewebe messen kann. Das ist im Allgemeinen nicht ganz einfach, da die Permittivität von vielen Faktoren abhängt und von Patient zu Patient stark variieren kann. Hierfür ist der Mikrowellenapplikator zunächst zu kalibrieren, sodass die Permittivität basierend auf den gemessenen Signalen bestimmt werden kann.

Bisherige Kalibrierungsmethoden erlauben den Mikrowellenapplikator basierend auf bekannte Materialstandards zu kalibrieren (siehe C. Hancock, N. Dharmasiri, M. White, and A. Goodman ,"The Design and Development of an Intergrated Multi- Functional Microwave Antenna Structure for Biological Applications", IEEE Transactions on Microwave Theory and Techniques, Vol.61, No.5, Mai 2013). Eine andere bekannte Kalibrierungsmöglichkeit besteht in der Nutzung von separaten Kalibrierungseinheiten, in die der Mikrowellenapplikator beispielsweise eingeführt wird; siehe z.B. US 2016/128 602 A2 oder EP 2 069 014 B1. Diese Kalibrierungen sind einerseits sehr aufwendig. Andererseits sind die Ergebnisse nicht sofort nutzbar, da noch Anpassungen an das speziell zu behandelnde Gewebe erforderlich sind. Dies erfolgt in der Regel über Nachschlagetabellen (look up table), die die einzelnen Permitivitäten für die jeweiligen Gewebe eines Patienten enthalten.

Ferner offenbart US2008234574 eine Kalibrierungsmethode wobei die Kalibrierung des Mikrowellenapplikators basierend auf Messungen an zumindest zwei vorbestimmten Materialien mit bekannten Permittivitäten durchgeführt wird.

Somit sind die erzielbaren Ergebnisse hinsichtlich der genauen Feststellung des Umrisses von malignem Gewebe entweder noch unzureichend oder aber nur aufwendig zu erhalten. Es besteht daher ein Bedarf nach verbesserten Kalibrierungsmöglichkeiten für Mikrowellenapplikatoren zur Behandlung von malignem Gewebe.

Kurzbeschreibung der Erfindung

Zumindest ein Teil der oben genannten Probleme wird durch eine Vorrichtung zur Kalibrierung eines Mikrowellenapplikators nach Anspruch 1 gelöst. Die abhängigen Ansprüche beziehen sich auf vorteilhafte Weiterbildungen der Vorrichtung nach Anspruch 1

Die vorliegende Erfindung bezieht sich auf eine Vorrichtung zur Kalibrierung eines Mikrowellenapplikators, der zur Behandlung von Gewebe geeignet ist. Die Vorrichtung umfasst: eine Steuereinrichtung zum Steuern von Kalibrierungssignalen, die zur Kalibrierung an den Mikrowellenapplikator anzulegen sind, eine Einrichtung zum Feststellen einer Umgebung zumindest eines Teils des Mikrowellenapplikators (z.B. eines Bereiches, der zur Behandlung genutzt wird) und eine Auswerteeinheit. Die Auswerteeinheit ist ausgebildet, um auf das Anlegen der Kalibrierungssignale zugehörige Reflexionssignale von der Umgebung des Mikrowellenapplikators zu bestimmen und die Kalibrierung des Mikrowellenapplikators basierend auf entsprechende Messungen an zumindest zwei vorbestimmten Umgebungen (Standards) mit bekannten Permittivitäten und basierend auf einer Messung in einer Umgebung mit gesundem Gewebe durchzuführen. Die Messung an dem gesunden Gewebe erfolgt automatisch auf ein Feststellen (durch die Einrichtung zum Feststellen), d.h. im Allgemeinen ohne eine Wechselwirkung mit dem Nutzer.

Ausführungsbeispiele definieren somit eine Vorrichtung für eine relative Kalibrierung des Mikrowellenapplikators, und zwar relativ zu einem bestimmten gesunden Gewebe. Um den Rand eines malignen Gewebes feststellen zu können, ist eine solche relative Kalibrierung in der Regel ausreichend. Eine absolute Kalibrierung wird im Allgemeinen nicht benötigt, da die Absolutwerte für das Erkennen eines Randes von malignem Gewebe nicht zwingend gebraucht werden. So soll die Vorrichtung in der Lage sein, relativ zu dem gesunden Gewebe einen Sprung in der Permittivität mit einer Auflösung festzustellen, die für malignem Gewebe typisch ist (zum Beispiel Sprünge um 5% oder um zumindest 10%). Für eine solche relative Kalibrierung sind gemäß Ausführungsbeispielen bereits zwei Messungen an Standards ausreichend, wenn zusätzlich als Referenzwert das gesunde Gewebe vermessen wird.

Es versteht sich, dass die genutzte Kalibrierung bzw. die Vorrichtung zur Kalibrierung nicht auf einen bestimmten Mikrowellenapplikator eingeschränkt sein soll. Vielmehr können Ausführungsbeispiele auf beliebige Applikatoren angewandt werden, zumindest solange die Behandlung auf die dielektrischen Eigenschaften des Gewebes basiert bzw. diese nutzt.

Optional ist die Steuereinrichtung ausgebildet, um vor einem Feststellen der Umgebung aus gesundem Gewebe Kalibrierungssignale für eine Luftumgebung an den Mikrowellenapplikator anzulegen. Die Auswerteeinheit ist optional weiter ausgebildet, um Reflexionssignale für die Luftumgebung zur Kalibrierung zu nutzen. Die Messung an eine Luftumgebung kann beispielsweise unmittelbar nach einem Einschalten der Vorrichtung durchgeführt werden. Ebenso ist es jedoch möglich, dass dazu eine explizite Nutzereingabe erfolgt, auf die die Vorrichtung die Kalibrierung an eine Luftumgebung durchführt.

Optional umfasst die Einrichtung eine Nutzerschnittstelle (z.B. ein Eingabeelement), die es dem Nutzer ermöglicht, zumindest eine der folgenden Eingaben durchzuführen:

- Feststellen eines Kontaktes mit gesundem Gewebe,
- Feststellen eines Kontaktes mit der ersten vorbestimmten Umgebung,
- Feststellen eines Kontaktes mit der zweiten vorbestimmten Umgebung,
- Feststellen eines Luftkontaktes,
- Starten der Kalibrierung,
- Beenden der Kalibrierung.

Optional umfasst die Vorrichtung weiter einen (Daten-) Speicher, um die Permittivitäten der vorbestimmten Umgebungen und/oder der Luft zu speichern. Die vorbestimmten Umgebungen können insbesondere Salzlösungen mit unterschiedlichen Salzkonzentrationen sein. Zum Beispiel können die zwei vorbestimmten Umgebungen zwei NaCl Lösungen, beispielsweise 2 mol/l und 4 mol/l oder aus dem dazwischenliegenden Bereich, umfassen. Optional können auch andere oder weitere NaCl Lösungen gewählt werden, beispielsweise aus den Lösungen von 2,0 mol/l bis 3,5 mol/l in beispielhaften 0,5 mol/l Schritten. Die gewählte Lösung (Salzkonzentration) kann an das zu erwartende menschliche Gewebe angepasst werden. Beispielsweise kann ein Standard höher, der andere Standard kleiner gewählt werden als der erwartete Wert des menschlichen Gewebes.

Optional umfassen die Kalibrierungssignale Mikrowellensignale von zumindest einer Frequenz. Die Reflexionssignale können Reflexionsfaktoren oder Streuparameter (z.B. Sn) der Umgebung sein oder darauf basieren. Die Mikrowellensignale können eine Vielzahl von Mikrowellensignalen in einem Frequenzbereich beispielsweise zwischen 2 GHz und 12 GHz umfassen (oder zwischen 5 GHz und 6 GHz oder zwischen 8 GHz und 10 GHz). Die Mikrowellensignale können für alle Umgebungen gleich oder unterschiedlich gewählt werden, wobei nacheinander verschiedene Frequenzen in dem gegebenen Bereich angelegt werden können.

Optional ist die Auswerteeinheit ausgebildet, um die Kalibrierung unter Nutzung einer Möbiustransformation durchzuführen, die durch folgende Formel gegeben ist:

$$\varepsilon_n = (a * \Gamma_n + b)/(c * \Gamma_n + d) , \qquad\qquad (1)$$

wobei $\varepsilon_n$ die Permittivitäten der verschiedenen Umgebungen, $\Gamma_n$ die zugehörige Reflexionsfaktoren, a, b, c, d während der Kalibrierung zu bestimmende Koeffizienten darstellen, die die Bedingung $a*d-b*c=1$ erfüllen, und n=1,2, ... die entsprechende Messung in Bezug auf die verschiedenen Umgebungen kennzeichnet. Es versteht sich, dass im Allgemeinen alle Parameter komplexe Werte annehmen.

Optional ist die Auswerteeinheit weiter ausgebildet, um nach erfolgter Kalibrierung einen Randbereich zwischen dem gesunden Gewebe und dem zu behandelndem Gewebe basierend auf einer Mindeständerung der ermittelten Permittivität zu ermitteln.

Die vorliegende Erfindung bezieht sich auch auf einen Mikrowellenapplikator zur Behandlung von Gewebe. Der Mikrowellenapplikator umfasst ein stabförmiges Behandlungselement, in welchem eine Koaxialleitung ausgebildet ist, um Mikrowellen zur Behandlung des Gewebes an eine Spitze des Behandlungsstabes weiterzuleiten. Außerdem umfasst der Mikrowellenapplikator eine der zuvor beschriebenen Vorrichtungen, die an die Koaxialleitung koppelt, um Mikrowellensignale und Kalibrierungssignale dem stabförmigen Behandlungselement zuzuführen und Reflexionssignale zu empfangen.

Optional umfasst die Koaxialleitung einen Innenleiter und einen Außenleiter. Der Außenleiter kann einen Spalt nahe der Spitze aufweisen. Außerdem kann das stabförmige Behandlungselement einen Metallzylinder in einem Abstand von einem Viertel einer Wellenlänge eines Mikrowellensignals in einer der Umgebungen des Mikrowellenapplikators umfassen, um eine rückwärtige Ausbreitung der Mikrowellen von der Spitze des stabförmigen Behandlungselementes weg zu unterdrücken.

Im Vergleich zu den bekannten Verfahren basieren Ausführungsbeispiele der vorliegenden Erfindung insbesondere auf eine Nutzung von gesundem Gewebe als weitere Informationsquelle, die zur Kalibrierung des Mikrowellenapplikators genutzt wird. Dadurch wird der Kontrast zwischen gesunden und dem malignen Gewebe deutlicher dargestellt. Dazu kann vor dem Beginn der eigentlichen Behandlung, und zwar beim Einführen des Mikrowellenapplikators, eine Messung an gesundem Gewebe durchgeführt werden, die die Kalibrierung des Mikrowellenapplikators abschließt und sofort automatisch eine patientenspezifische Kalibrierung des Mikrowellenapplikators erreicht. Da malignes Gewebe häufig von gesundem Gewebe umgeben ist, kann die Kalibrierung daher während des Eingriffes abgeschlossen werden. Wenn beispielsweise ein Organ wie die Leber malignes Gewebe aufweist, kann der Mikrowellenapplikator über einen gesunden Randbereich der beispielhaften Leber eingeführt werden und zur Kalibrierung genutzt werden. Durch die Nutzung des zu durchstoßenden gesunden Gewebes zur Kalibrierung kann eine deutliche Verbesserung des Kontrastes zwischen vorliegendem gesundem und malignem Gewebe erreicht werden. Ausführungsbeispiele lösen somit zumindest einen Teil der eingangs erwähnten Probleme durch eine relative Kalibrierung basierend auf die Detektion von Permittivitätsänderungen, die beim Übergang von gesundem und malignem Gewebe auftreten und zum Beispiel in einem Bereich zwischen 15 % und 30 % liegen. Die genaue Position des Mikrowellenapplikators relativ zu dem Tumorgewebe kann damit deutlich genauer ermittelt werden, als dies bei konventionellen Verfahren möglich ist. Beispielsweise kann der Mikrowellenapplikator über einen Computertomografen oder ein MRT relativ zu dem Tumorgewebe bildlich sehr gut dargestellt werden. Die relative Permitivitätsmessung erlaubt es den Rand des malignen Gewebes deutlich darzustellen.

Ausführungsbeispiele bieten somit im Vergleich zu den bekannten Mikrowellenapplikatoren den Vorteil, dass der Arzt mehr Information während und nach der Ablation erhält. Ebenso ist es möglich, die Mikrowellenbehandlung ständig zu verfolgen, da die dielektrischen Eigenschaften des Gewebes direkt durch den Applikator gemessen werden und der Arzt daher stets genau weiß, wo der Mikrowellenapplikator relativ zu dem Rand des malignen Gewebes sich befindet.

Kurzbeschreibung der Figuren

Die Ausführungsbeispiele der vorliegenden Erfindung werden besser verstanden anhand der folgenden detaillierten Beschreibung und den beiliegenden Zeichnungen der unterschiedlichen Ausführungsbeispiele, die jedoch nicht so verstanden werden sollten, dass sie die Offenbarung auf die spezifischen Ausführungsformen einschränken, sondern lediglich der Erklärung und dem Verständnis dienen.

Fig. 1      zeigt eine Vorrichtung zur Kalibrierung eines Mikrowellenapplikators gemäß einem Ausführungsbeispiel der vorliegenden Erfindung.

Fig. 2      veranschaulicht beispielhaft die Vorgehensweise der Kalibrierung, wie sie durch die Vorrichtung automatisch ausgeführt wird.

Fig. 3A,3B      zeigen weitere Details des Mikrowellenapplikators gemäß Ausführungsbeispielen der vorliegenden Erfindung.

Fig. 4      veranschaulicht eine Frequenzabhängigkeit einer gemessenen Permittivität (z.B. dessen Realteil) nach der Kalibrierung.

Detaillierte Beschreibung

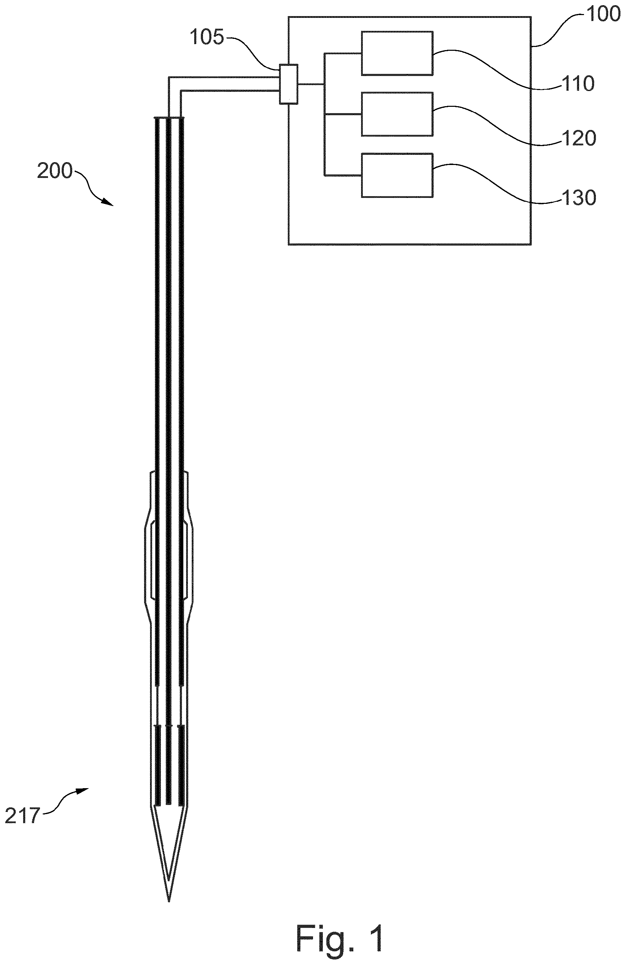

**Fig. 1** zeigt eine Vorrichtung 100 gemäß einem Ausführungsbeispiel der vorliegenden Erfindung, die zur Kalibrierung eines Mikrowellenapplikators 200 geeignet ist, wobei der Mikrowellenapplikator 200 zur Behandlung von Gewebe mittels einer Mikrowellenbehandlung nutzbar ist. Die Vorrichtung 100 umfasst eine Steuereinrichtung 110 zum Steuern von Kalibrierungssignalen (z.B. deren Erzeugung mit vorgegebenen Frequenz und Intensität), die zur Kalibrierung des Mikrowellenapplikators 200 anzulegen sind. Die Vorrichtung umfasst weiter eine Einrichtung 120 zum Feststellen eines Kontaktes des Mikrowellenapplikators 200 mit einem gesunden Gewebe. Diese Einrichtung 120 kann beispielsweise eine Anzeige aufweisen, um dem Nutzer Informationen zur Kalibrierung zu geben. Die Anzeige kann - nach der Kalibrierung - auch gleich den Rand des malignen Gewebes grafisch darstellen. Schließlich umfasst die Vorrichtung 100 eine Auswerteeinheit 130, die in der Lage ist, um Reflexionssignale, die auf das Anlegen der Kalibrierungssignale von den Mikrowellenapplikator 200 zurückgesandt werden, zu bestimmen oder zu erfassen und darauf basierend die Kalibrierung durchzuführen.

Der Mikrowellenapplikator 200 ist im Allgemeinen nicht Teil der Vorrichtung 100, sondern kann über einen Anschluss 105 mit der Vorrichtung 100 verbunden werden. Außerdem wird der Mikrowellenapplikator 200 in der Regel nicht vollständig in die jeweilige zu messende Umgebung gebracht, sondern lediglich die Spitze 217 oder ein Bereich der Spitze 217.

Zur Kalibrierung des Mikrowellenapplikators 200 werden Messungen an zumindest zwei vorbestimmten Lösungen bzw. an einer Lösung und an Luft mit bekannten Permittivitäten durchgeführt. Zusätzlich ist die Auswerteeinheit ausgebildet, um für die Kalibrierung eine Messung an einem gesunden Gewebe zu nutzen, wobei die Messung an dem gesunden Gewebe auf ein Feststellen der Einrichtung 120, dass ein entsprechender Kontakt vorliegt, automatisch erfolgt (ohne eine Wechselwirkung mit einem Nutzer).

Die Einrichtung 120 zum Feststellen kann auch eine Detektionseinrichtung sein oder umfassen, die automatisch Kontakte zu einer bestimmten Umgebung detektiert oder auf eine Eingabe des Nutzers hin feststellt. Beispielsweise kann die Einrichtung 120 eine Nutzerschnittstelle umfassen, die es dem Nutzer ermöglicht, über ausdrückliche Eingaben Kontakte zu bestimmten Umgebungen festzustellen. Dazu gehören insbesondere: Feststellen eines Kontaktes mit gesundem Gewebe, Feststellen eines Kontaktes mit der ersten vorbestimmten Umgebung, Feststellen eines Kontaktes mit der zweiten vorbestimmten Umgebung, Feststellen eines Luftkontaktes. Diese Schnittstelle kann auch zum Starten der Kalibrierung und/oder zum Beenden der Kalibrierung genutzt werden. Auf das Starten kann beispielswiese automatisch die Messung an Luft ausgeführt werden.

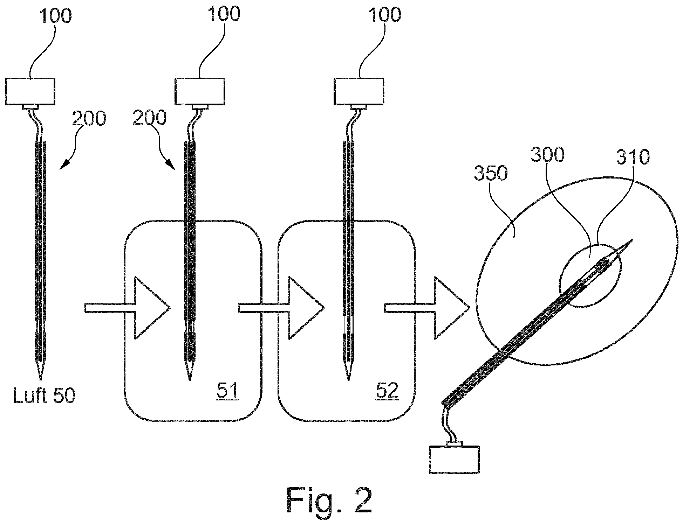

**Fig. 2** veranschaulicht beispielhaft die Vorgehensweise der Kalibrierung, wie sie durch die Vorrichtung 100 automatisch ausgeführt wird. So kann beispielsweise zunächst in einer Luftumgebung 50 des Mikrowellenapplikators 200 eine Messung durchgeführt werden. Daran anschließend kann der Mikrowellenapplikator 200 in ein erstes Medium 51 (z.B. eine Flüssigkeit) eingetaucht, Kalibrierungssignale ausgesandt und anschließend Reflexionssignale empfangen und ausgewertet werden. Daran anschließend kann optional ein zweites Medium 52 (z.B. auch eine Flüssigkeit) genutzt werden, um auch dort Kalibrierungssignale einzuleiten und die Reflexionssignale zur Kalibrierung zu nutzen. Schließlich wird die Kalibrierung dadurch abgeschlossen, dass der Mikrowellenporator 200 auf einen Kontakt mit gesundem Gewebe 350 die Kalibrierung automatisch abschließt. Es versteht sich, dass insgesamt drei Messungen ausreichen, wovon eine am gesunden Gewebe 350 durchgeführt wird, während die anderen beiden an zwei Flüssigkeiten 51, 52 oder an einer Flüssigkeit und an Luft 50 durchgeführt werden.

Ein besonderer Vorteil von Ausführungsbeispielen der vorliegenden Erfindung besteht darin, dass für den Fall, dass das maligne Gewebe 300 innerhalb von gesunde Gewebe 350 sich befindet, die Kalibrierung automatisch auf dem Weg zur Behandlung des malignem Gewebes 300 abgeschlossen werden kann, da der Mikrowellenapplikator 200 durch das gesunde Gewebe 350 zu führen ist und dabei die Messung an dem gesunden Gewebe 350 durchgeführt werden kann.

Nach der Kalibrierung kann der Mikrowellenapplikator 200 genutzt werden, um beispielsweise das maligne Gewebe 300 in dem gesunden Gewebe 350 zu behandeln (z.B. für die Mikrowellenablation). Für diese Behandlung ist es insbesondere wichtig, dass der Rand 310 des malignen Gewebes 300 möglichst genau festgestellt wird, sodass der Arzt in der Lage ist, festzustellen, ob die Spitze 217 des Mikrowellenporator 200, die zur Behandlung genutzt wird, sich in malignem Gewebe 300 oder gesundem Gewebe 350 befindet.

Beispielsweise können für das erste und zweite Medium 51, 52 Salzwasserlösungen mit folgenden Salzkonzentrationen genutzt werden: für die erste Flüssigkeit 51 eine Konzentration von 0,2 mol/l (oder 1 mol/l oder 2 mol/1), für die zweite Flüssigkeit 52 eine Konzentration von 4 mol/l. Optional können auch andere oder weitere NaCl Lösungen gewählt werden, beispielsweise Konzentrationen von 2,0 mol/l und/oder 2,5 mol/l und/oder 3,0 mol/l und/oder 3,5 mol/1.

Für diese Medien und für Luft sind die Permittivitäten $\varepsilon_n$ (n=i,2, ..) bekannt, die über eine Möbiustransformation $\varepsilon_n = (a*\Gamma_n+b)/(c*\Gamma_n+d)$ mit die zugehörigen Reflexionsfaktoren $\Gamma_n$ zusammenhängen, wobei die Koeffizienten a, b, c, d unter der Bedingung $a*d-b*c=1$ durch mehrere Messungen gefunden werden können. Hierzu ist ein Gleichungssystem zu lösen, was durch die Auswerteeinheit 130 durchgeführt werden kann. Die drei unbekannten Koeffizienten könnten drei Messungen an Medien ermittelt werden. Hierauf basieren die bekannten Kalibrierungsverfahren.

Ausführungsbeispiele brauchen jedoch nur zwei Messungen an bekannten Medien 51, 52. Mit der Messung an gesundem Gewebe 350, ist es möglich, die Bestimmung der Koeffizienten a, b, c, d bis auf den Permittivitätswert des gesunden Gewebes 350 durchzuführen. Da zur Detektierung des Randes 310 nur Sprünge in der Permittivität zu ermitteln sind, ist die Kenntnis des Absolutwertes der Permittivität des gesunden Gewebes 350 nicht erforderlich. Daher ist die Kalibrierung gemäß Ausführungsbeispielen einfacher umzusetzen, wobei als gesundes Gewebe automatisch jenes Gewebe genutzt werden kann, in dem der Tumor eingebettet ist (dessen ungefähre Lage kann zuvor bestimmt werden).

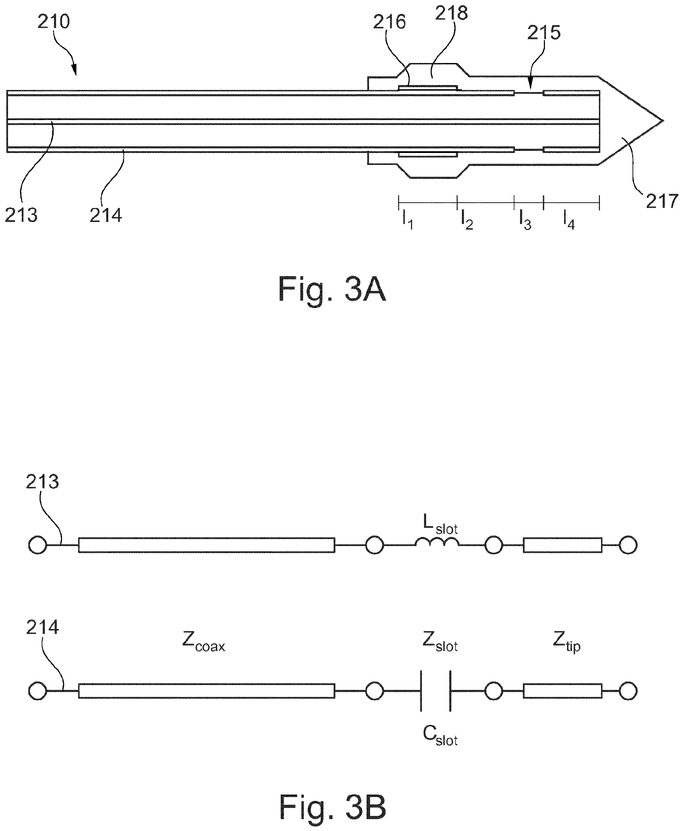

**Fig. 3A** zeigt weitere Details eines möglichen Mikrowellenapplikators gemäß Ausführungsbeispielen der vorliegenden Erfindung. Der Mikrowellenapplikator 200 umfasst eine Koaxialleitung mit einem Innenleiter 213 und einem Außenleiter 214, wobei an einem Ende ein Anschluss zu der Vorrichtung 100 vorgesehen ist und an einem gegenüberliegenden Ende die Koaxialleitung in einer Spitze 217 endet. Die Koaxialleitung ist dabei innerhalb eines stabförmigen Behandlungselementes 210 ausgebildet, welches für die Behandlung des malignen Gewebes 300 geeignet ist. Entlang des stabförmigen Behandlungselementes 210 ist ein Metallzylinder 216 und ein Spalt 215 in dem Außenleiter 214 vorgesehen. Der Spalt 215 dient als eine Öffnung für die Mikrowellenstrahlung aus dem Inneren des Koaxialleiters. Der Metallzylinder 216 ist in einer Entfernung l2 von dem Spalt 215 angeordnet, wobei die Entfernung l2 einen Wert von ungefähr einem Viertel einer Wellenlänge der Mikrowellenstrahlung innerhalb des Gewebes entspricht. Damit wird erreicht, dass die Ausbreitung der Mikrowellenstrahlung weg von der Spitze 217 unterdrückt wird und somit eine effiziente Behandlung des malignen Gewebes 350 ermöglicht wird. Der Metallzylinder 216 und/oder der Spalt 215 erstreckt sich zylinderförmiger um den Außenleiter 214 herum.

Außerdem ist in dem Behandlungsbereich an der Spitze 217 eine Verkleidung 118 vorgesehen, die den Bereich des Spaltes 215 und den Bereich des Metallzylinders 216 abdeckt. Somit wird ein direkter Kontakt des Metallzylinders 216 bzw. des Außenleiters 214 mit dem umgebenden Gewebe verhindert. Die Abdeckung 218 kann beispielsweise ein PTFE Material aufweisen. Die axiale Länge des Metallzylinders 216 umfasst beispielsweise einen Wert l1 von beispielsweise ca. 3 mm, die Entfernung l2 umfasst beispielsweise einen Wert von ca. 4 mm und der Spalt 115 umfasst beispielsweise eine axiale Ausdehnung l3 von ca. 1,5 mm. Die Entfernung des Spaltes 115 von dem Endbereich des Koaxialkabels kann beispielsweise eine Entfernung $l_4$ aufweisen, die gleich gewählt werden kann, wie die Entfernung $l_2$.

**Fig. 3B** zeigt ein Ersatzschaltbild für die Koaxialleitung entlang des Behandlungselementes 210. In dem Ersatzschaltbild wird ersichtlich, dass die Koaxialleitung eine Impedanz $Z_{coax}$ aufweist, der Spaltabschnitt eine Induktivitäten $L_{slot}$ und eine Kapazität $C_{slot}$ aufweist, die zu einer Impedanz $Z_{slot}$ führt. Die Spitze umfasst ebenfalls eine Impedanz $Z_{tip}$. Alle Impedanzen überlagern sich. Wenn Kalibrierungssignale auf den Mikrowellenapplikator 200 geleitet werden, kommt es zu Reflexionen, da die Impedanzen im Allgemeinen nicht mit den Impedanzen der verschiedenen Umgebungen angepasst sein werden. Die Stärke der Reflexionen hängt von den dielektrischen Eigenschaften des umgebenden Gewebes bzw. des umgebenden Mediums ab, sodass aus den Reflexionskoeffizienten die Permittivitäten des umgebenden Mediums bestimmt werden können.

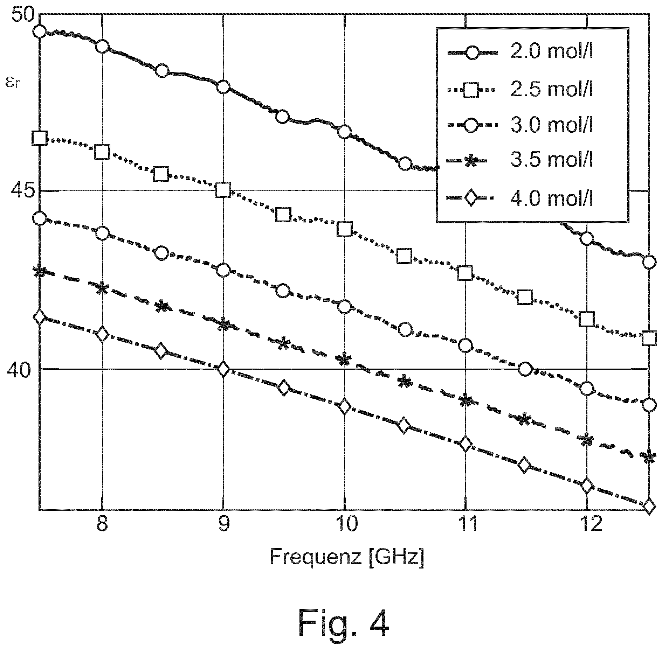

**Fig. 4** veranschaulicht eine Abhängigkeit der Permittivität $\varepsilon_r$ oder dessen Realteil von der Frequenz der Kalibrierungssignale und gibt verschiedene Graphen für unterschiedliche Salzkonzentrationen an. Aus der Fig. 4 ist ersichtlich, dass Permittivitätsunterschiede von 15 % deutlich aufgelöst werden können. Somit wird es möglich, den Kontrast des malignen Gewebes 300, wo es zu Änderungen von zumindest 15 % kommt, eindeutig detektiert werden können. Ausführungsbeispiele ermöglichen somit, den Rand 310 des malignen Gewebes 300 innerhalb des gesunden Gewebes 350 eindeutig festzustellen.

Bezugszeichenliste

| | |
|---|---|
| 50 | Luftumgebung |
| 51, 52 | Flüssigkeitsumgebungen |
| 100 | Vorrichtung |
| 110 | Steuereinrichtung |
| 120 | Detektionseinrichtung |
| 130 | Auswerteeinheit |
| 200 | Mikrowellenapplikator |
| 210 | stabförmige Behandlungselement |
| 213 | Innenleiter |
| 214 | Außenleiter |
| 215 | Spalt |
| 216 | Metallzylinder |
| 217 | Spitze |
| 218 | Abdeckung |
| 300 | zu behandelndes (malignes) Gewebe |
| 310 | Rand des malignem Gewebes |
| 350 | gesundes Gewebe |

**Patentansprüche**

1. Vorrichtung (100) zur Kalibrierung eines Mikrowellenapplikators (200) zur Behandlung von Gewebe (300), mit folgenden Merkmalen:

   eine Steuereinrichtung (110) zum Steuern von Kalibrierungssignalen, die zur Kalibrierung an den Mikrowellenapplikator (200) anzulegen sind;

eine Einrichtung (120) zum Feststellen einer Umgebung (51, 52, 350) zumindest eines Teils des Mikrowellenapplikators (200);

**gekennzeichnet durch**:

eine Auswerteeinheit (130), die ausgebildet ist, um auf das Anlegen der Kalibrierungssignale zugehörige Reflexionssignale ($\Gamma_n$) von der Umgebung (51, 52, 350) des Mikrowellenapplikators (200) zu bestimmen und die Kalibrierung des Mikrowellenapplikators (200) basierend auf Messungen an zumindest zwei vorbestimmten Umgebungen mit bekannten Permittivitäten ($\varepsilon_n$) und basierend auf einer Messung in einer Umgebung mit gesundem Gewebe (350) durchzuführen, wobei die Messung an dem gesunden Gewebe (350) auf ein Feststellen durch die Einrichtung (120) automatisch erfolgt.

2. Vorrichtung (100) nach Anspruch 1, wobei die Steuereinrichtung (110) ausgebildet ist, um vor einem Feststellen der Umgebung aus dem gesunden Gewebe (350) Kalibrierungssignale für eine Luftumgebung (50) an den Mikrowellenapplikator (200) anzulegen und die Auswerteeinheit (130) weiter ausgebildet ist, um Reflexionssignale ($\Gamma_n$) für die Luftumgebung (51) zur Kalibrierung zu nutzen.

3. Vorrichtung (100) nach Anspruch 1 oder Anspruch 2, wobei die Einrichtung (120) eine Nutzerschnittstelle umfasst, die dem Nutzer ermöglicht, zumindest eine der folgenden Eingaben durchzuführen:

- Feststellen eines Kontaktes mit gesundem Gewebe,
- Feststellen eines Kontaktes mit der ersten vorbestimmten Umgebung,
- Feststellen eines Kontaktes mit der zweiten vorbestimmten Umgebung,
- Feststellen eines Luftkontaktes,
- Starten der Kalibrierung,
- Beenden der Kalibrierung.

4. Vorrichtung (100) nach einem der vorhergehenden Ansprüche, die weiter einen Speicher aufweist, um die Permittivitäten der vorbestimmten Umgebungen und/oder der Luft zu speichern, wobei die vorbestimmten Umgebungen insbesondere Salzlösungen mit unterschiedlichen Salzkonzentrationen sind und zumindest eine NaCl Lösung aus einem Bereich zwischen 0,2 mol/l und 4 mol/l umfassen.

5. Vorrichtung (100) nach einem der vorhergehenden Ansprüche, wobei die Kalibrierungssignale Mikrowellensignale von zumindest einer Frequenz umfassen und die Reflexionssignale ($\Gamma_n$) durch Reflexionsfaktoren der Umgebung gegeben sind.

6. Vorrichtung (100) nach Anspruch 5, wobei die Mikrowellensignale eine Vielzahl von Mikrowellensignalen in einem Frequenzbereich zwischen 2 GHz und 12 GHz oder zwischen 5 GHz und 6 GHz oder zwischen 8 GHz und 10 GHz umfassen.

7. Vorrichtung (100) nach einem der vorhergehenden Ansprüche, wobei die Auswerteeinheit (130) ausgebildet ist, um die Kalibrierung unter Nutzung einer Möbiustransformation durchzuführen, die durch folgende Formel gegeben ist:

$$\varepsilon_n = (a^*\Gamma_n + b)/(c^*\Gamma_n + d) \, ,$$

wobei $\varepsilon_n$ die Permittivitäten der verschiedenen Umgebungen sind, $\Gamma_n$ die zugehörige Reflexionsfaktoren sind, a, b, c, d während der Kalibrierung zu bestimmende Koeffizienten darstellen, die die Bedingung a*d-b*c=1 erfüllen, und n=1,2, ... die entsprechende Messung an den verschiedenen Umgebungen (51, 52, 350) kennzeichnet.

8. Vorrichtung (100) nach einem der vorhergehenden Ansprüche, wobei die Auswerteeinheit (130) weiter ausgebildet ist, um nach erfolgter Kalibrierung einen Randbereich (310) zwischen dem gesunden Gewebe (350) und dem zu behandelndem Gewebe (300) basierend auf einer Mindeständerung einer ermittelten Permittivität ($\varepsilon$) zu bestimmen.

9. Mikrowellenapplikator (200) zur Behandlung von Gewebe (300), mit:

einem stabförmigen Behandlungselement (210), in welchem eine Koaxialleitung (213, 214) ausgebildet ist, um Mikrowellen zur Behandlung des Gewebes (300) an eine Spitze (217) des Behandlungsstabes (210) weiterzuleiten; und

einer Vorrichtung (100) nach einem der vorhergehenden Ansprüche, die an die Koaxialleitung (213, 214) koppelt, um Mikrowellensignale und Kalibrierungssignale dem stabförmigen Behandlungselement (210) zuzuführen und Reflexionssignal ($\Gamma_n$) zu empfangen.

10. Mikrowellenapplikator (200) nach Anspruch 9, wobei

die Koaxialleitung einen Innenleiter (213) und einen Außenleiter (214) umfasst und der Außenleiter (214) einen Spalt (215) nahe der Spitze (217) aufweist, und

das stabförmige Behandlungselement (210) einen Metallzylinder (216) in einem Abstand ($l_2$) von einem Viertel einer Wellenlänge eines Mikrowellensignals in einer der Umgebungen des Mikrowellenapplikators (200) umfasst, um eine rückwärtige Ausbreitung der Mikrowellen von der Spitze (217) des stabförmigen Behandlungselementes (210) weg zu unterdrücken.

**Claims**

1. Apparatus (100) for calibrating a microwave applicator (200) for treating tissue (300), comprising the following features:

a controller (110) for controlling calibration signals which are to be applied to the microwave applicator (200) for calibration;

a device (120) for identifying an environment (51, 52, 350) of at least part of the microwave applicator (200);

**characterized by:**

an evaluation unit (130) which is designed, upon application of the calibration signals, to determine associated reflection signals ($\Gamma_n$) from the environment (51, 52, 350) of the microwave applicator (200), and to calibrate the microwave applicator (200) based on measurements at at least two predetermined environments with known permittivities ($\varepsilon_n$) and based on a measurement in a healthy tissue environment (350), the measurement at the healthy tissue (350) being automatically performed upon identification by the device (120).

2. Apparatus (100) according to claim 1, wherein the controller (110) is designed to apply calibration signals for an air environment (50) to the microwave applicator (200) before identifying the healthy tissue environment (350), and the evaluation unit (130) is further designed to use reflection signals ($\Gamma_n$) for the air environment (51) for calibration.

3. Apparatus (100) according to either claim 1 or claim 2, wherein the device (120) comprises a user interface allowing the user to perform at least one of the following inputs:

- establishing contact with healthy tissue,
- establishing contact with the first predetermined environment,
- establishing contact with the second predetermined environment,
- establishing air contact,
- starting the calibration,
- terminating the calibration.

4. Apparatus (100) according to any of the preceding claims, further comprising a memory to store the permittivities of the predetermined environments and/or of the air, wherein the predetermined environments are in particular salt solutions having different salt concentrations, and at least one NaCl solution from a range between 0.2 mol/l and 4 mol/l.

5. Apparatus (100) according to any of the preceding claims, wherein the calibration signals comprise microwave signals of at least one frequency and the reflection signals ($\Gamma_n$) are given by reflection factors of the environment.

6. Apparatus (100) according to claim 5, wherein the microwave signals comprise a plurality of microwave signals in a frequency range between 2 GHz and 12 GHz, or between 5 GHz and 6 GHz, or between 8 GHz and 10 GHz.

7. Apparatus (100) according to any of the preceding claims, wherein the evaluation unit (130) is designed to perform the calibration using a Möbius transformation given by the following formula:

$$\varepsilon_n = (a*\Gamma_n+b)/(c*\Gamma_n+d),$$

wherein $\varepsilon_n$ are the permittivities of the different environments, $\Gamma_n$ are the associated reflection factors, a, b, c, d represent coefficients to be determined during calibration, which meet the condition a*d-b*c=1, and n=1,2, ... characterizes the corresponding measurement at the various environments (51, 52, 350).

8. Apparatus (100) according to any of the preceding claims, wherein the evaluation unit (130) is further designed, after calibration has taken place, to identify an edge region (310) between the healthy tissue (350) and the tissue (300) to be treated based on a minimum change of a detected permittivity ($\varepsilon$).

9. Microwave applicator (200) for treating tissue (300), comprising:

a rod-shaped treatment element (210) in which a coaxial line (213, 214) is formed to forward microwaves for treating the tissue (300) to a tip (217) of the treatment rod (210); and
an apparatus (100) according to any of the preceding claims, which is coupled to the coaxial line (213, 214) in order to supply microwave signals and calibration signals to the rod-shaped treatment element (210) and receive reflection signal ($\Gamma_n$).

10. Microwave applicator (200) according to claim 9, wherein

the coaxial line comprises an inner conductor (213) and an outer conductor (214), and the outer conductor (214) has a gap (215) near the tip (217), and
the rod-shaped treatment element (210) comprises a metal cylinder (216) which is at a distance (l2) from a quarter of a wavelength of a microwave signal in one of the environments of the microwave applicator (200), in order to suppress a rearward spread of the microwaves away from the tip (217) of the rod-shaped treatment element (210).

## Revendications

1. Dispositif (100) permettant l'étalonnage d'un applicateur micro-ondes (200) pour le traitement de tissus (300), comportant les caractéristiques suivantes :

un appareil de commande (110) permettant de commander des signaux d'étalonnage qui sont appliqués à l'applicateur micro-ondes (200) pour l'étalonnage ;
un appareil (120) permettant de détecter un environnement (51, 52, 350) d'au moins une partie de l'applicateur micro-ondes (200) ;
**caractérisé par** :
une unité d'évaluation (130) configurée pour déterminer des signaux de réflexion ($\Gamma_n$) provenant de l'environnement (51, 52, 350) de l'applicateur micro-ondes (200) et associés à l'application des signaux d'étalonnage et pour réaliser l'étalonnage de l'applicateur micro-ondes (200) sur la base de mesures au niveau d'au moins deux environnements prédéterminés comportant des permittivités ($\varepsilon_n$) connues et sur la base d'une mesure dans un environnement comportant du tissu sain (350), la mesure au niveau du tissu sain (350) étant effectuée automatiquement par le dispositif (120) lors d'une détection.

2. Dispositif (100) selon la revendication 1, dans lequel l'appareil de commande (110) est configuré pour appliquer des signaux d'étalonnage pour un environnement d'air (50) à l'applicateur micro-ondes (200) avant une détection de l'environnement à partir du tissu sain (350) et l'unité d'évaluation (130) est en outre configurée pour utiliser des signaux de réflexion ($\Gamma_n$) pour l'environnement d'air (51) pour l'étalonnage.

3. Dispositif (100) selon la revendication 1 ou la revendication 2, dans lequel l'appareil (120) comprend une interface utilisateur qui permet à l'utilisateur de réaliser au moins l'une des entrées suivantes :

- détection d'un contact avec du tissu sain,
- détection d'un contact avec le premier environnement prédéterminé,
- détection d'un contact avec le second environnement prédéterminé,
- détection d'un contact avec l'air,

- démarrage de l'étalonnage,
- fin de l'étalonnage.

4. Dispositif (100) selon l'une des revendications précédentes, présentant en outre une mémoire pour stocker les permittivités des environnements prédéterminés et/ou de l'air, dans lequel les environnements prédéterminés sont en particulier des solutions salines comportant différentes concentrations de sel et comprennent au moins une solution de NaCl dans une plage comprise entre 0,2 mol/l et 4 mol/l.

5. Dispositif (100) selon l'une des revendications précédentes, dans lequel les signaux d'étalonnage comprennent des signaux micro-ondes d'au moins une fréquence et les signaux de réflexion ($\Gamma_n$) sont donnés par des facteurs de réflexion de l'environnement.

6. Dispositif (100) selon la revendication 5, dans lequel les signaux micro-ondes comprennent une pluralité de signaux micro-ondes dans une plage de fréquences comprise entre 2 GHz et 12 GHz ou entre 5 GHz et 6 GHz ou entre 8 GHz et 10 GHz.

7. Dispositif (100) selon l'une des revendications précédentes, dans lequel l'unité d'évaluation (130) est configurée pour réaliser l'étalonnage en utilisant une transformation de Möbius donnée par la formule suivante :

$$\varepsilon_n = (a * \Gamma_n + b)/(c * \Gamma_n + d),$$

où $\varepsilon_n$ représente les permittivités des différents environnements, $\Gamma_n$ représente les facteurs de réflexion associés, a, b, c, d représentent des coefficients à déterminer pendant l'étalonnage qui répondent à la condition $a * d - b * c = 1$, et $n = 1, 2, \ldots$ et indique la mesure correspondante pour les différents environnements (51, 52, 350).

8. Dispositif (100) selon l'une des revendications précédentes, dans lequel l'unité d'évaluation (130) est en outre configurée pour, après l'étalonnage réalisé, déterminer une zone périphérique (310) entre le tissu sain (350) et le tissu à traiter (300) sur la base d'une variation minimale d'une permittivité ($\varepsilon$) déterminée.

9. Applicateur micro-ondes (200) permettant le traitement de tissus (300), comportant :

un élément de traitement en forme de tige (210) dans lequel une ligne coaxiale (213, 214) est configurée pour transmettre des micro-ondes pour le traitement du tissu (300) à une pointe (217) de la tige de traitement (210) ; et
un dispositif (100) selon l'une des revendications précédentes, lequel est couplé à la ligne coaxiale (213, 214) pour envoyer des signaux micro-ondes et des signaux d'étalonnage à l'élément de traitement en forme de tige (210) et pour recevoir des signaux de réflexion ($\Gamma_n$).

10. Applicateur micro-ondes (200) selon la revendication 9, dans lequel

la ligne coaxiale comprend un conducteur intérieur (213) et un conducteur extérieur (214) et le conducteur extérieur (214) présente une fente (215) à proximité de la pointe (217), et
l'élément de traitement en forme de tige (210) comprend un cylindre métallique (216) à une distance (l2) d'un quart d'une longueur d'onde d'un signal micro-ondes dans l'un des environnements de l'applicateur micro-ondes (200) afin d'atténuer une propagation arrière des micro-ondes à l'écart de la pointe (217) de l'élément de traitement en forme de tige (210).

Fig. 1

Fig. 2

Fig. 3A

Fig. 3B

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2016128602 A2
- EP 2069014 B1

- US 2008234574 A

**In der Beschreibung aufgeführte Nicht-Patentliteratur**

- **C. HANCOCK ; N. DHARMASIRI ; M. WHITE ; A. GOODMAN.** The Design and Development of an Intergrated Multi- Functional Microwave Antenna Structure for Biological Applications. *IEEE Transactions on Microwave Theory and Techniques,* 05. Mai 2013, vol. 61